# EUROPEAN PATENT SPECIFICATION

(11) **EP 4 243 150 B1**
(45) Date of publication and mention of the grant of the patent: **10.12.2025**
(21) Application number: 22895873.2
(22) Date of filing: 21.10.2022
(51) Int. Cl.: H01M 10/0567, H01M 10/052, H01M 4/525, H01M 4/505, H01M 4/36, H01M 4/587, H01M 4/38, H01M 4/48, H01M 4/58, C07C 311/15, C07D 207/36, C07D 233/84, C07D 307/64

(54) **NOVEL ADDITIVE FOR NON-AQUEOUS ELECTROLYTE SOLUTION AND LITHIUM SECONDARY BATTERY COMPRISING SAME**
NEUARTIGES ADDITIV FÜR NICHTWÄSSRIGE ELEKTROLYTLÖSUNG UND LITHIUMSEKUNDÄRBATTERIE ES ENTHALTEND
NOUVEL ADDITIF POUR SOLUTION ÉLECTROLYTIQUE NON AQUEUSE ET BATTERIE SECONDAIRE AU LITHIUM LE COMPRENANT

(30) Priority: 16.11.2021 KR 20210157227
(43) Date of publication of application: 13.09.2023
(73) Proprietor: LG Energy Solution, Ltd., Seoul 07335 (KR)
(72) Inventor: JEONG, You Kyeong, Daejeon 34122 (KR); AHN, Kyoung Ho, Daejeon 34122 (KR); HAN, Jun Hyeok, Daejeon 34122 (KR); SHIN, Won Kyung, Daejeon 34122 (KR); LEE, Won Tae, Daejeon 34122 (KR); JI, Su Hyeon, Daejeon 34122 (KR); OH, Young Ho, Daejeon 34122 (KR)
(74) Representative: Hoffmann Eitle
(86) International application number: PCT/KR2022/016110
(87) International publication number: WO 2023/090665

(56) References cited:
- EP-A1- 3 261 166
- WO-A1-2022/069385
- JP-A- 2000 082 494
- KR-A- 20160 079 574
- KR-A- 20170 021 335
- KR-A- 20190 025 693
- KR-A- 20190 025 693
- KR-A- 20210 052 812
- US-A- 4 510 324
- US-A1- 2002 009 650
- US-A1- 2017 204 124
- US-A1- 2021 135 290
- US-B2- 11 050 083

## Description

### [Technical Field]

The present invention relates to a novel additive for a non-aqueous electrolyte and a lithium secondary battery including the same.

This application claims the benefit of a priority based on Korean Patent Application No. 10-2021-0157227, filed on November 16, 2021.

### [Background]

Recently, secondary batteries are widely applied not only to small devices such as portable electronic devices, but to medium and large devices such as battery packs or power storage devices of hybrid or electric vehicles. Examples of these secondary batteries may include non-aqueous electrolyte batteries such as lithium-ion batteries, lithium batteries, lithium-ion capacitors, and sodium ion batteries.

Among these non-aqueous electrolyte batteries, lithium-ion batteries are used by injecting an electrolyte into a battery cell including a positive electrode including a positive electrode active material that enables intercalation and deintercalation of lithium and a negative electrode including a negative electrode active material that enables intercalation and deintercalation of lithium. Particularly, an electrolyte uses an organic solvent in which a lithium salt is dissolved, and it is important to determine the stability and performance of a lithium secondary battery.

For example, LiPF₆, which is the most widely used lithium salt for an electrolyte, reacts with an electrolyte solvent to promote the depletion of the solvent and generate HF. The HF generated thereby may not only generate a large amount of gas under a high temperature condition, but also elute metal ions, and when the eluted metal ions are generated in the form of a precipitate on the surface of a negative electrode, it causes an increase in potential of a negative electrode and a drop in cell open-circuit voltage (OCV), leading to problems such as degraded battery performance as well as a reduction in lifespan and high-temperature safety.

EP 3 261 166 describes an electrolyte solution comprising a non-aqueous solvent, a solute, at least one silane compound represented by the general formula (1) as a first compound, and a fluorine-containing compound represented by the general formula (3), for example, as a second compound.

US 20170204124 describes an ionic complex, an electrolyte for nonaqueous electrolyte battery, a nonaqueous electrolyte battery and an ionic complex synthesis method, wherein the ionic complex is represented by any of formulae (1) to (3).

JP2000082494 describes a flame-retardant non-aqueous electrolyte used for electrochemical devices such as secondary batteries, primary batteries, and electric double layer capacitors, and is particularly suitable for secondary batteries.

### [Description of the Invention]

### [Technical Problem]

Therefore, the present invention is directed to providing a developed technology of forming a coating film on an electrode surface to prevent direct contact between a positive electrode and an electrolyte, inhibit gas generation by lowering the oxidative decomposition of the electrolyte while blocking direct contact between the positive electrode, HF, PF₅, etc. and improve a capacity retention rate and prevent the OCV drop of a battery by improving a metal ion precipitation phenomenon of the positive electrode.

### [Technical Solution]

To solve the above-described problem,
one embodiment of the present invention provides
an electrolyte additive for a secondary battery, which includes
any one or more compounds selected from Structural Formulae 2, 3, 4, 5, 6, 7, 10, 11, 12, 13, 14, 15, 16, 17, 18, 19, 20, 21, 22, 23, 24, 25, 26, 27, 28, 30, 31, 32, 33, 34 and 35:

| | | |
|---|---|---|
| | Structural Formula 2 | Structural Formula 3 |
| | 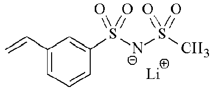 | 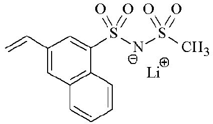 |
| Structural Formula 4 | Structural Formula 5 | Structural Formula 6 |
| 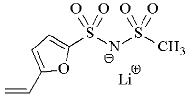 | 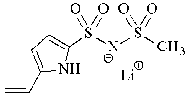 | 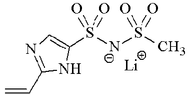 |
| Structural Formula 7 | | |
| 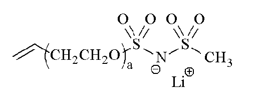 | | |
| Structural Formula 10 | Structural Formula 11 | Structural Formula 12 |
| 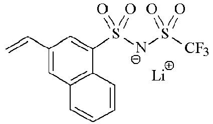 | 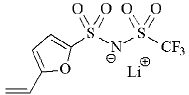 | 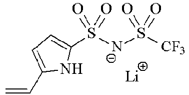 |
| Structural Formula 13 | Structural Formula 14 | Structural Formula 15 |
| 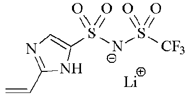 | 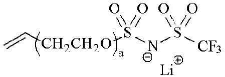 | 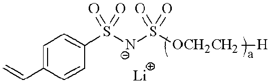 |
| Structural Formula 16 | Structural Formula 17 | Structural Formula 18 |
| 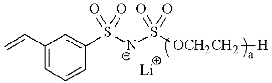 | 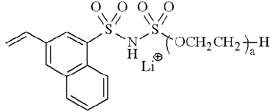 | 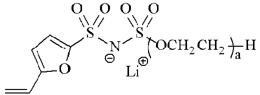 |
| Structural Formula 19 | Structural Formula 20 | Structural Formula 21 |
| 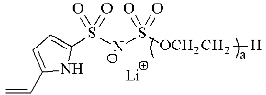 | 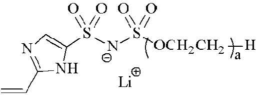 | 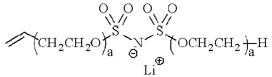 |
| Structural Formula 22 | Structural Formula 23 | Structural Formula 24 |
| 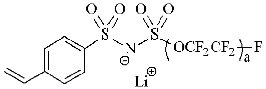 | 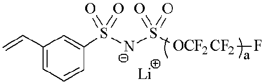 | 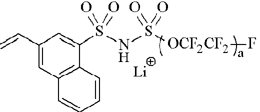 |
| Structural Formula 25 | Structural Formula 26 | Structural Formula 27 |
| 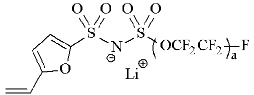 | 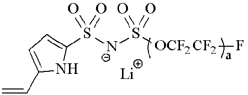 | 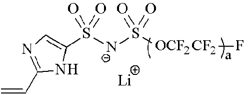 |
| Structural Formula 28 | | Structural Formula 30 |
| 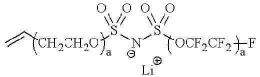 | | 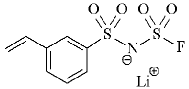 |
| Structural Formula 31 | Structural Formula 32 | Structural Formula 33 |
| 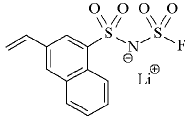 | 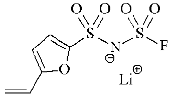 | 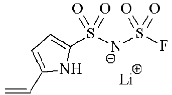 |
| Structural Formula 34 | Structural Formula 35 | |
| 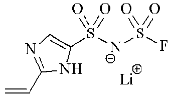 | 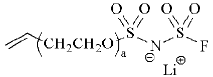 | |

wherein, in Structural Formula 7 and Structural Formula 14 to 28, a is an integer of 2 to 5.

In addition, one embodiment of the present invention provides
an electrolyte composition for a lithium secondary battery, which includes a non-aqueous organic solvent; a lithium salt; and a compound
being any one or more compounds selected from Structural Formulae 2, 3, 4, 5, 6, 7, 10, 11, 12, 13, 14, 15, 16, 17, 18, 19, 20, 21, 22, 23, 24, 25, 26, 27, 28, 30, 31, 32, 33, 34 and 35:

| | | |
|---|---|---|
| | Structural Formula 2 | Structural Formula 3 |
| | 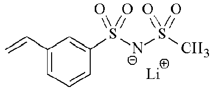 | 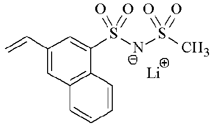 |
| Structural Formula 4 | Structural Formula 5 | Structural Formula 6 |
| 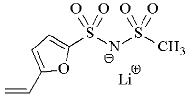 | 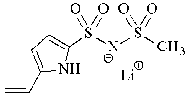 | 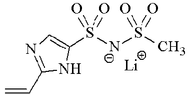 |
| Structural Formula 7 | | |
| 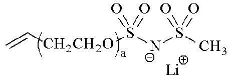 | | |
| Structural Formula 10 | Structural Formula 11 | Structural Formula 12 |
| 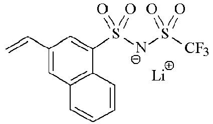 | 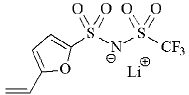 | 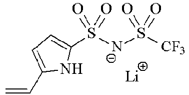 |
| Structural Formula 13 | Structural Formula 14 | Structural Formula 15 |
| 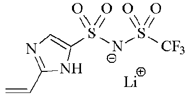 | 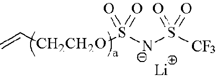 | 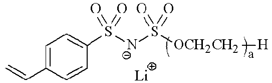 |
| Structural Formula 16 | Structural Formula 17 | Structural Formula 18 |
| 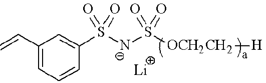 | 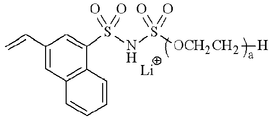 | 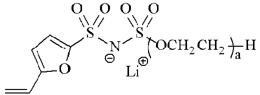 |
| Structural Formula 19 | Structural Formula 20 | Structural Formula 21 |
| 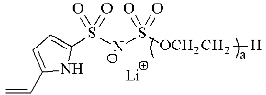 | 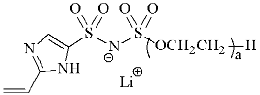 | 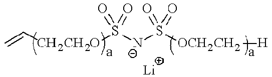 |
| Structural Formula 22 | Structural Formula 23 | Structural Formula 24 |
| 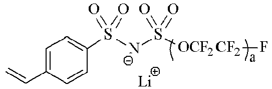 | 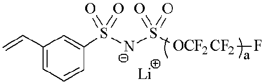 | 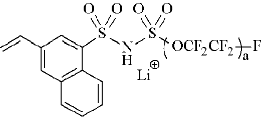 |
| Structural Formula 25 | Structural Formula 26 | Structural Formula 27 |
| 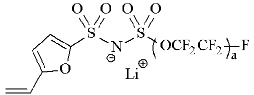 | 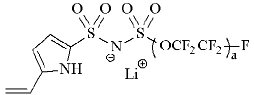 | 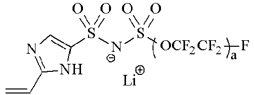 |
| Structural Formula 28 | | Structural Formula 30 |
| 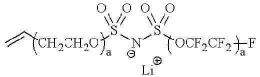 | | 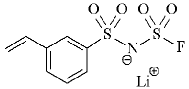 |
| Structural Formula 31 | Structural Formula 32 | Structural Formula 33 |
| 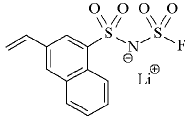 | 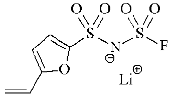 | 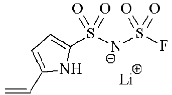 |
| Structural Formula 34 | Structural Formula 35 | |
| 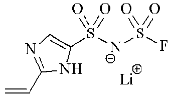 | 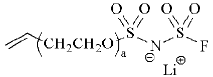 | |

wherein, in Structural Formula 7 and Structural Formula 14 to 28, a is an integer of 2 to 5.

Here, the compound represented by any one of the Formulae 2-7, 10-28 and 30-35 may be included at 0.01 to 3 wt% with respect to the total weight of the electrolyte composition.

In addition, the lithium salt may include one or more selected from the group consisting of LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, and (FSO₂)₂NLi.

In addition, the non-aqueous organic solvent may include N-methyl-2-pyrrolidinone, ethylene carbonate, propylene carbonate, butylene carbonate, dimethyl carbonate, diethyl carbonate, γ-butyrolactone, 1,2-dimethyoxy ethane, tetrahydrofuran, 2-methyl tetrahydrofuran, dimethyl sulfoxide, 1,3-dioxolane, formamide, dimethylformamide, dioxolane, acetonitrile, nitromethane, methyl formate, methyl citrate, phosphoric acid triester, trimethoxy methane, a dioxolane derivative, sulfolane, methyl sulfolane, 1,3-dimethyl-2-imidazolidinone, a propylene carbonate derivative, a tetrahydrofuran derivative, ether, methyl propionate, and ethyl propionate.

Furthermore, one embodiment of the present invention provides a lithium secondary battery, which includes:
an electrode assembly that includes a positive electrode including one or more positive electrode active materials of lithium metal oxides represented by Formulas 2 and 3 below, a negative electrode, and a separator interposed between the positive electrode and the negative electrode; and
the electrolyte composition according to the present invention:

   [Formula 2] Liₓ[Ni_{y}Co_{z}Mn_{w}M¹ᵥ]O₂

   [Formula 3] LiM²ₚMn₍₂₋ₚ₎O₄

In Formulas 2 and 3,
M¹ is one or more elements selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo,
x, y, z, w and v satisfy 1.0≤x≤1.30, 0.5≤y<1, 0<z≤0.3, 0<w≤0.3, and 0≤v≤0.1, respectively, wherein y+z+w+v=1,
M² is Ni, Co or Fe, and
p is 0.05≤p≤0.6.

Here, the positive electrode active material may include one or more selected from the group consisting of LiNi_{0.8}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}Co_{0.2}Mn_{0.15}Al_{0.05}O₂, LiNi_{0.7}Co_{0.1}Mn_{0.1}Al_{0.1}O₂, and LiNi_{0.5}Mn_{1.5}O₄.

In addition, the negative electrode active material may consist of a carbon material and a silicon material, wherein the silicon material may include one or more of silicon (Si), silicon carbide (SiC) and silicon oxide (SiO_{q}, 0.8≤q≤2.5).

Moreover, the silicon material may be included at 1 to 20 wt% with respect to the total weight of the negative electrode active material.

### [Advantageous Effects]

An electrolyte additive according to the present invention forms a coating film on an electrode surface in the activation of a secondary battery, thereby the generation of a large amount of gas under a high temperature condition can be prevented, and a cell OCV drop and a decrease in capacity retention rate, which are caused by elution of metal ions from an electrode, can be effectively prevented, resulting in effective improvement in durability, performance and high-temperature safety of the battery.

### [Brief Description of the Drawings]

FIG. 1 is a graph showing the results of Raman spectroscopy of electrolyte compositions respectively prepared in Example 1 and Comparative Example 1 according to the present invention.
FIG. 2 is a graph showing the results of differential capacity curve analysis for half-cells including the electrolyte compositions respectively prepared in Example 1 and Comparative Example 1 according to the present invention.
FIG. 3 is a graph showing the results of linear sweep voltammetry for three-electrode batteries including the electrolyte compositions respectively prepared in Example 1 and Comparative Example 1 according to the present invention.
FIG. 4 and FIG.5 are a set of graphs showing the results of X-ray photoelectron spectroscopy for coating films formed on positive and negative electrodes surfaces of each lithium secondary battery including the electrolyte composition prepared in Example 9, Comparative Example 15 and Comparative Example 19 respectively, after charging/discharging according to the present invention.

### [Best Mode for Carrying Out the invention]

The present invention may have various modifications and various examples, and thus specific examples are illustrated in the drawings and described in detail in the detailed description.

However, it should be understood that the present invention is not limited to specific embodiments, and includes all alternatives which fall within the scope of the appended claims.

The terms "comprise," "include" and "have" used herein designate the presence of characteristics, numbers, steps, actions, components or members described in the specification or a combination thereof, and it should be understood that the possibility of the presence or addition of one or more other characteristics, numbers, steps, actions, components, members or a combination thereof is not excluded in advance.

In addition, when a part of a layer, a film, a region or a plate is disposed "on" another part, this includes not only a case in which one part is disposed "directly on" another part, but a case in which a third part is interposed therebetween. In contrast, when a part of a layer, a film, a region or a plate is disposed "under" another part, this includes not only a case in which one part is disposed "directly under" another part, but a case in which a third part is interposed therebetween. In addition, in this application, "on" may include not only a case of disposed on an upper part but also a case of disposed on a lower part.

In addition, in the present invention, the "included as a main component" may mean that a defined component is included at 50 wt% or more, 60 wt% or more, 70 wt% or more, 80 wt% or more, 90 wt% or more, or 95 wt% or more with respect to the total weight. For example, the "graphite is included as a main ingredient in a negative electrode active material" means that graphite is included at 50 wt% or more, 60 wt% or more, 70 wt% or more, 80 wt% or more, 90 wt% or more, or 95 wt% or more with respect to the total weight of the negative electrode active material, and in some cases, it means that a negative electrode active material totally consists of graphite and thus includes 100 wt% of graphite.

Hereinafter, the present invention will be described in further detail.

### Electrolyte additive for secondary battery

One embodiment of the present invention provides
an electrolyte additive for a secondary battery including
any one or more compounds selected from Structural Formulae 2, 3, 4, 5, 6, 7, 10, 11, 12, 13, 14, 15, 16, 17, 18, 19, 20, 21, 22, 23, 24, 25, 26, 27, 28, 30, 31, 32, 33, 34 and 35:

| | | |
|---|---|---|
| | Structural Formula 2 | Structural Formula 3 |
| | 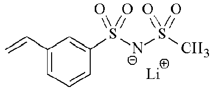 | 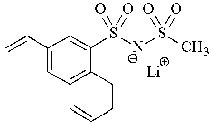 |
| Structural Formula 4 | Structural Formula 5 | Structural Formula 6 |
| 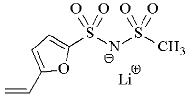 | 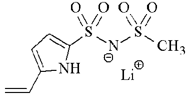 | 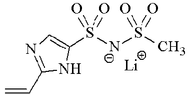 |
| Structural Formula 7 | | |
| 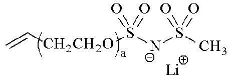 | | |
| Structural Formula 10 | Structural Formula 11 | Structural Formula 12 |
| 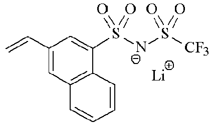 | 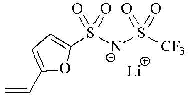 | 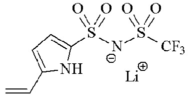 |
| Structural Formula 13 | Structural Formula 14 | Structural Formula 15 |
| 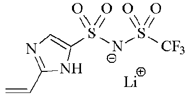 | 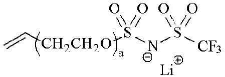 | 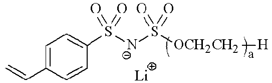 |
| Structural Formula 16 | Structural Formula 17 | Structural Formula 18 |
| 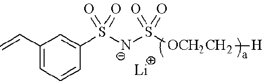 | 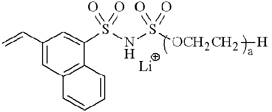 | 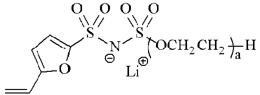 |
| Structural Formula 19 | Structural Formula 20 | Structural Formula 21 |
| 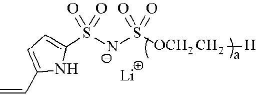 | 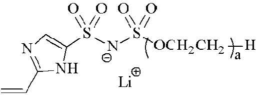 | 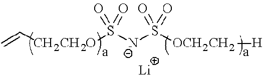 |
| Structural Formula 22 | Structural Formula 23 | Structural Formula 24 |
| 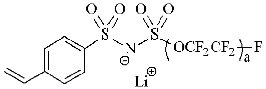 | 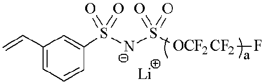 | 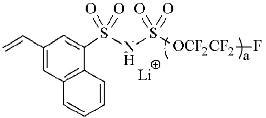 |
| Structural Formula 25 | Structural Formula 26 | Structural Formula 27 |
| 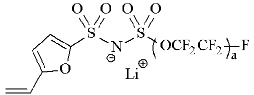 | 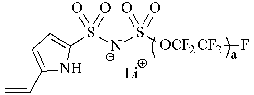 | 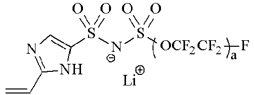 |
| Structural Formula 28 | | Structural Formula 30 |
| 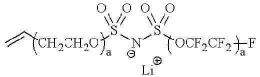 | | 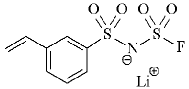 |
| Structural Formula 31 | Structural Formula 32 | Structural Formula 33 |
| 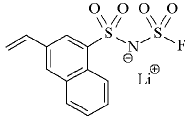 | 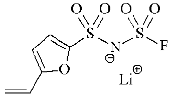 | 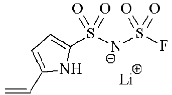 |
| Structural Formula 34 | Structural Formula 35 | |
| 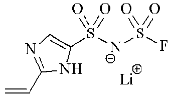 | 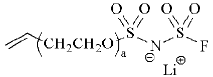 | |

wherein, in Structural Formula 7 and Structural Formula 14 to 28, a is an integer of 2 to 5.

The electrolyte additive for a secondary battery according to the present invention includes an ionic compound including a mother nucleus to which a vinyl group is bonded via a functional group that extends an amide conjugation or a functional group that has a polyglycolide unit cell at one side based on a sulfonylimide group as shown in the Formulae 2-7, 10-28 and 30-35. Since the vinyl group is bonded to one side based on a sulfonylimide group to form the conjugation or bonded via polyglycolide unit cell, the compound has a structure that has a charge in the molecule. Therefore, an organic and/or inorganic coating film may be uniformly formed on the surface of the positive electrode and/or the negative electrode in the activation of the secondary battery including the electrolyte additive. That is, the electrolyte additive may be directly contained in an organic and/or inorganic film disposed on the surface of the positive electrode and/or the negative electrode in the form of a monomolecule (or monomer) when the secondary battery is activated. Through this, when the battery is exposed to a high temperature, it is possible to inhibit gas generation caused by the decomposition of the electrolyte and improve the OCV drop phenomenon of the battery which occur at the positive electrode.

The electrolyte additive according to the present invention, as described above, since the vinyl group is bonded to one side based on a sulfonylimide group via a functional group that extends a conjugation of a sulfonylimide group or a functional group that has a polyglycolide unit cell, has a structure that has a charge in the molecule. Therefore, the electrolyte additive may be directly involved in a solvation shell of lithium ions even under a low potential in the activation of the battery to uniformly form an inorganic coating film through a reduction reaction on the surface of the negative electrode and to uniformly form an inorganic coating film through an oxidation reaction on the surface of the positive electrode at the same time.

Therefore, the electrolyte additive can inhibit gas generation caused by decomposition of the electrolyte when the battery is exposed to a high temperature, and improve the OCV drop phenomenon and capacity reduction of the battery, which occur at the positive electrode, resulting in the prevention of the deterioration of a secondary battery and further improvement in high-temperature safety.

### Electrolyte composition for lithium secondary battery

In addition, one embodiment of the present invention provides
an electrolyte composition for a lithium secondary battery, which includes a non-aqueous organic solvent; a lithium salt; and a compound being any one or more compounds selected from Structural Formulae 2, 3, 4, 5, 6, 7, 10, 11, 12, 13, 14, 15, 16, 17, 18, 19, 20, 21, 22, 23, 24, 25, 26, 27, 28, 30, 31, 32, 33, 34 and 35:

| | | |
|---|---|---|
| | Structural Formula 2 | Structural Formula 3 |
| | 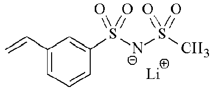 | 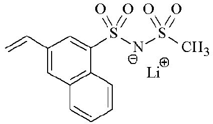 |
| Structural Formula 4 | Structural Formula 5 | Structural Formula 6 |
| 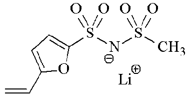 | 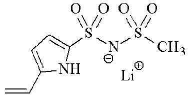 | 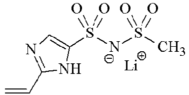 |
| Structural Formula 7 | | |
| 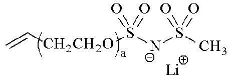 | | |
| Structural Formula 10 | Structural Formula 11 | Structural Formula 12 |
| 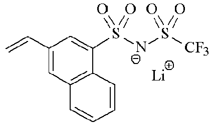 | 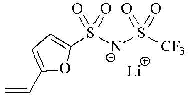 | 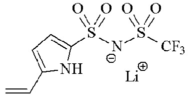 |
| Structural Formula 13 | Structural Formula 14 | Structural Formula 15 |
| 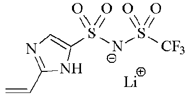 | 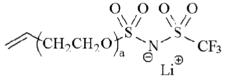 | 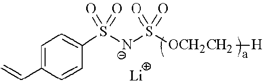 |
| Structural Formula 16 | Structural Formula 17 | Structural Formula 18 |
| 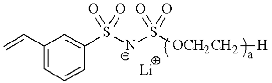 | 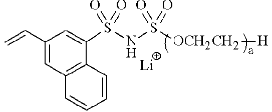 | 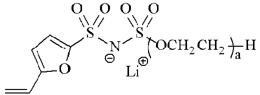 |
| Structural Formula 19 | Structural Formula 20 | Structural Formula 21 |
| 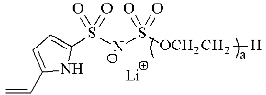 | 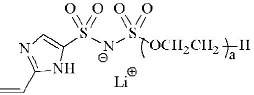 | 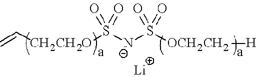 |
| Structural Formula 22 | Structural Formula 23 | Structural Formula 24 |
| 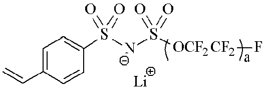 | 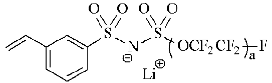 | 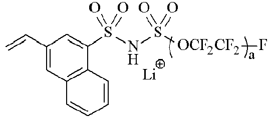 |
| Structural Formula 25 | Structural Formula 26 | Structural Formula 27 |
| 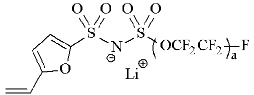 | 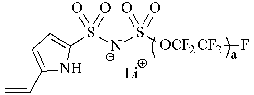 | 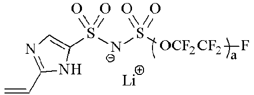 |
| Structural Formula 28 | | Structural Formula 30 |
| 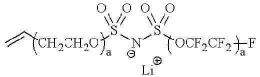 | | 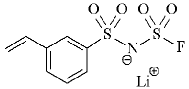 |
| Structural Formula 31 | Structural Formula 32 | Structural Formula 33 |
| 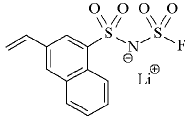 | 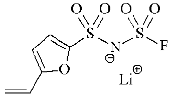 | 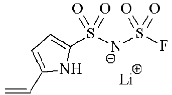 |
| Structural Formula 34 | Structural Formula 35 | |
| 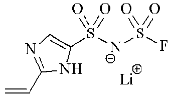 | 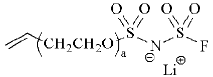 | |

wherein, in Structural Formula 7 and Structural Formula 14 to 28, a is an integer of 2 to 5.

Here, the compound represented by any one of the Formulae 2-7, 10-28 and 30-35 may be included at a certain content in the electrolyte composition. Specifically, the compound represented by any one of the Formulae 2-7, 10-28 and 30-35 may be included at 0.01 to 5 wt%, and more specifically, 0.05 to 3 wt% or 1.0 to 2.5 wt% with respect to the total weight of the electrolyte composition. When the electrolyte additive is used in an excess amount outside the above range, the viscosity of the electrolyte composition is increased thereby deteriorating of the wettability of the electrode and separator. Furthermore, a polymerization by self-reaction is induced in the electrolyte composition, thereby resulting in poor battery performance such as a decrease in the initial capacity of the battery, due to a reduction in ion conductivity of the electrolyte composition. In addition, the present invention may prevent the additive effect from being insignificantly implemented when the electrolyte additive is used at a trace amount outside the above range.

Meanwhile, the lithium salt used in the electrolyte composition may be applied without particular limitation as long as it is used in a non-aqueous electrolyte in the art. Specifically, the lithium salt may include one or more selected from the group consisting of LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, and (FSO₂)₂NLi.

The concentration of the lithium salt is not particularly limited, and the lower limit of the appropriate concentration range is 0.5 mol/L or more, specifically 0.7 mol/L or more, and more specifically 0.9 mol/L or more, and the upper limit of the appropriate concentration range is 2.5 mol/L or less, specifically 2.0 mol/L or less, and more specifically 1.5 mol/L or less. When the concentration of the lithium salt is lower than 0.5 mol/L, there is a risk that ion conductivity is reduced, and the cycle characteristics and output characteristics of a non-aqueous electrolyte battery are lowered. In addition, when the concentration of the lithium salt exceeds 2.5 mol/L, the viscosity of an electrolyte for non-aqueous electrolyte battery increases, thereby there is a risk of decreasing ion conductivity and lowering the cycle characteristics and output characteristics of a non-aqueous electrolyte battery.

In addition, when a large amount of lithium salt is dissolved in a non-aqueous organic solvent at one time, the liquid temperature may increase because of the dissolution heat for the lithium salt. As described above, when the temperature of the non-aqueous organic solvent significantly increases due to the dissolution heat for the lithium salt, there is a risk that the decomposition may be accelerated to generate hydrogen fluoride (HF). Hydrogen fluoride (HF) is not preferable because it causes degraded battery performance. Therefore, a temperature at which the lithium salt is dissolved in a non-aqueous organic solvent may be adjusted to -20 to 80 °C, and specifically 0 to 60 °C, but the present invention is not particularly limited thereto.

Furthermore, a non-aqueous organic solvent used in the electrolyte composition may be applied without particular limitation as long as it can be used in a non-aqueous electrolyte in the art. Specifically, examples of the non-aqueous organic solvents may include aprotic organic solvents such as N-methyl-2-pyrrolidinone, ethylene carbonate (EC), propylene carbonate, butylene carbonate, dimethyl carbonate, diethyl carbonate, γ-butyrolactone, 1,2-dimethyoxy ethane, tetrahydrofuran, 2-methyl tetrahydrofuran, dimethyl sulfoxide, 1,3-dioxolane, formamide, dimethylformamide, dioxolane, acetonitrile, nitromethane, methyl formate, methyl citrate, phosphoric acid triester, trimethoxy methane, a dioxolane derivative, sulfolane, methyl sulfolane, 1,3-diethyl-2-imidazolidinone, a propylene carbonate derivative, a tetrahydrofuran derivative, ether, methyl propionate, and ethyl propionate.

In addition, as a non-aqueous solvent used in the present invention, one type of the above examples may be used alone, or two or more types thereof may be used by mixing in any combination and ratio according to purpose. In terms of electrochemical stability against the oxidation/reduction of the solvent and chemical stability against heat or the reaction with a solute, among the above examples, particularly, propylene carbonate, ethylene carbonate, fluoroethylene carbonate, diethyl carbonate, dimethyl carbonate, or ethyl methyl carbonate is preferable.

Meanwhile, the electrolyte composition may further include an additive, other than the above-described basic components. Without departing the gist of the present invention, an additive generally used in the non-aqueous electrolyte of the present invention may be added at any ratio. Specifically, the additive may be a compound having an overcharge prevention effect, a negative electrode coating film-forming effect, and a positive electrode protection effect, such as cyclohexylbenzene, biphenyl, t-butylbenzene, vinylene carbonate, vinylethylene carbonate, difluoroanisole, fluoroethylene carbonate, propane sultone, succinonitrile, or dimethylvinylene carbonate. In addition, in the case of use in a non-aqueous electrolyte battery called a lithium polymer battery, it is also possible to use an electrolyte for a non-aqueous electrolyte battery after being pseudo-solidified by a gelling agent or crosslinked polymer.

### Lithium secondary battery

Further, one embodiment of the present invention provides a lithium secondary battery, which includes:
an electrode assembly that includes a positive electrode including one or more positive electrode active materials of lithium metal oxides represented by Formulas 2 and 3 below; a negative electrode; and a separator interposed between the positive electrode and the negative electrode; and
the electrolyte composition according to the present invention:

   [Formula 2] Liₓ[Ni_{y}Co_{z}Mn_{w}M¹ᵥ]O₂

   [Formula 3] LiM²ₚMn₍₂₋ₚ₎O₄

In the Formula 2 and Formula 3,
M¹ is one or more elements selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo,
x, y, z, w and v satisfy 1.0≤x≤1.30, 0.5≤y<1, 0<z≤0.3, 0<w≤0.3, and 0≤v≤0.1, respectively, wherein y+z+w+v=1,
M² is Ni, Co or Fe, and
p is 0.05≤p≤0.6.

The lithium secondary battery according to the present invention includes a positive electrode including a positive electrode active material, a negative electrode including a negative electrode active material, a separator interposed between the positive electrode and the negative electrode, and the lithium salt-containing non-aqueous electrolyte composition of the present invention, which is described above.

Specifically, the positive electrode includes a positive electrode mixture layer formed by applying, drying, and pressing a positive electrode active material on a positive electrode current collector, and may selectively further include a conductive material, a binder, or other additives as needed.

Here, the positive electrode active material is a material that can cause an electrochemical reaction on the positive electrode current collector and may include one or more lithium metal oxides represented by the Formula 2 and Formula 3, which enable reversible intercalation and deintercalation of lithium ions.

The lithium metal oxides represented by the Formula 2 and Formula 3 are materials containing nickel (Ni) and manganese (Mn) at high contents, respectively, and have advantages of stably supplying high capacity and/or high voltage electricity when used as a positive electrode active material. In addition, the activation of the secondary battery requires a high charging potential of about 4.0V or more to form a coating film on the surface of the positive electrode and/or the negative electrode. Unlike the conventional positive electrode active materials with a charging potential of less than 4.0V such as iron phosphate, the lithium metal oxides have a high charging potential of about 4.0 or more, and thus, a coating film may be formed easily on the electrode.

Here, examples of the lithium metal oxides represented by the Formula 2 may include LiNi_{0.8}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}Co_{0.2}Mn_{0.15}Al_{0.05}O₂, and LiNi_{0.7}Co_{0.1}Mn_{0.1}Al_{0.1}O₂, and examples of the lithium metal oxides represented by Formula 3 may include LiNi_{0.7}Mn_{1.3}O₄; LiNi_{0.5}Mn_{1.5}O₄; and LiNi_{0.3}Mn_{1.7}O₄, and these oxides may be used alone or in combination.

In addition, in the positive electrode, as a positive electrode current collector, a material that does not cause a chemical change in the corresponding battery and has high conductivity may be used. For example, stainless steel, aluminum, nickel, titanium, or calcined carbon may be used and in the case of aluminum or stainless steel, one that is surface treated with carbon, nickel, titanium or silver may also be used. In addition, the average thickness of the current collector may be suitably selected within 3 to 500 µm in consideration of the conductivity and total thickness of the positive electrode to be formed.

In addition, the negative electrode, like the positive electrode, includes a negative electrode mixture layer formed by applying, drying and pressing a negative electrode active material on a negative electrode current collector, and may selectively further include a conductive material, a binder, or other additives as needed.

The negative electrode active material may include a carbon material and a silicon material. Specifically, the carbon material refers to a material that has a carbon atom as the main component, and examples of the carbon materials may include one or more selected from the group consisting of natural graphite, artificial graphite, expanded graphite, non-graphitizing carbon, carbon black, acetylene black, and Ketjen black. In addition, the silicon material refers to a material that has a silicon atom as the main component, and may include silicon (Si), silicon carbide (SiC), silicon monoxide (SiO) or silicon dioxide (SiO₂) alone or in combination. When, as the silicon (Si)-containing materials, silicon monoxide (SiO) and silicon dioxide (SiO₂) are uniformly mixed or combined to be included in the negative electrode mixture layer, these materials may be represented as a silicon oxide (SiO_{q}, 0.8≤q≤2.5).

In addition, the silicon material may be included at 1 to 20 wt%, and specifically, 3 to 10 wt%, 8 to 15 wt%, 13 to 18 wt%, or 2 to 8 wt% with respect to the total weight of the negative electrode active material. The present invention may maximize the energy density of the battery by controlling the content of the silicon material in the above content range.

In addition, the negative electrode current collector is not particularly limited as long as it has high conductivity without causing a chemical change in the battery, and for example, copper, stainless steel, nickel, titanium or calcined carbon may be used, and in the case of copper or stainless steel, one whose surface is treated with carbon, nickel, titanium or silver may be used. Furthermore, the average thickness of the negative electrode current collector may be suitably selected within 1 to 500 µm in consideration of the conductivity and total thickness of the negative electrode to be formed.

Meanwhile, the separator interposed between the positive electrode and the negative electrode of each unit cell is an insulating thin film having high ion permeability and high mechanical strength, and is not particularly limited as long as it is one that is commonly used in the art. Specifically, the separator may include one or more polymers selected from chemical-resistant and hydrophobic polypropylene, polyethylene and a polyethylene-propylene copolymer. The separator may have the form of a porous polymer substrate, such as a sheet or non-woven fabric including the above-described polymer, and in some cases, have the form of a composite separator in which organic or inorganic particles on the porous polymer substrate are coated with an organic binder. In addition, the separator may have an average pore diameter of 0.01 to 10 µm, and an average thickness of 5 to 300 µm.

Further, the secondary battery includes the above-described non-aqueous electrolyte composition according to the present invention as an electrolyte.

The electrolyte composition, as an electrolyte additive, includes an ionic compound represented by any one or more compounds selected from Structural Formulae 2, 3, 4, 5, 6, 7, 10, 11, 12, 13, 14, 15, 16, 17, 18, 19, 20, 21, 22, 23, 24, 25, 26, 27, 28, 30, 31, 32, 33, 34 and 35, which has a mother nucleus to which a vinyl group is bonded via a functional group that extends an amide conjugation or a functional group that has a polyglycolide unit cell, at one side based on a sulfonylimide group:
Since the vinyl group is bonded to one side based on a sulfonylimide group to form an amide conjugation or bonded via polyglycolide unit cell, the compound has a structure that has a charge in the molecule. Therefore, an organic and/or inorganic coating film may be uniformly formed on the surface of the positive electrode and/or the negative electrode in the activation of the secondary battery including the electrolyte additive. To this end, the electrolyte additive may inhibit gas generation caused by the decomposition of the electrolyte when the battery is exposed to a high temperature, and improve the OCV drop phenomenon and capacity reduction of the battery, which occur at the positive electrode, resulting in further improvement in performance and high-temperature safety of the battery.

Hereinafter, the present invention will be described in further detail with reference to examples and experimental examples.

However, the following examples and experimental example merely illustrate the present invention, and the content of the present invention is not limited to the following examples and experimental examples.

### Examples 1 to 4 and Comparative Examples 1 to 6. Preparation of electrolyte composition for lithium secondary battery

A non-aqueous electrolyte composition was prepared by dissolving 1M concentration of LiPF₆ as a lithium salt in a solvent in which ethylene carbonate (EC) and ethyl methyl carbonate (EMC) were mixed in a volume ratio of 3:7, and dissolving an additive to be 2 wt% with respect to the total weight of the electrolyte as shown in Table 1 below.

**[Table 1]**

| | Type of non-aqueous electrolyte additive |
|---|---|
| Example 1 reference | 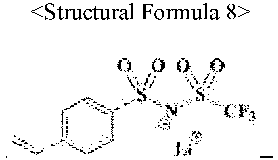 |
| Example 2 | 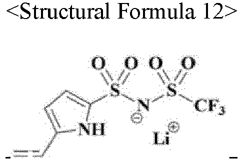 |
| Example 3 | 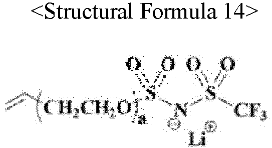 |
| Example 4 | 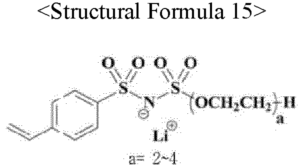 |
| Comparative Example 1 | Not added |
| Comparative Example 2 | 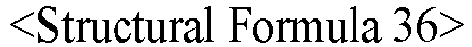 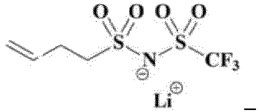 |
| Comparative Example 3 | 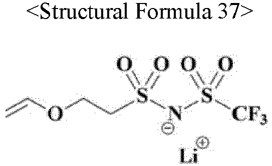 |
| Comparative Example 4 | 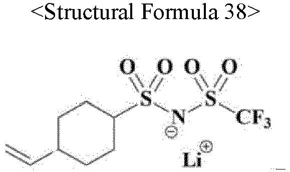 |
| Comparative Example 5 | 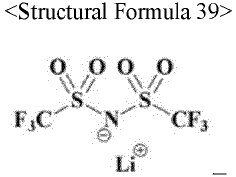 |
| Comparative Example 6 | 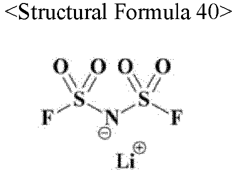 |

### Comparative Examples 7. Preparation of electrolyte composition for lithium secondary battery

As an electrolyte additive, a non-aqueous electrolyte composition for a lithium secondary battery was prepared in the same manner as in Example 1, except that an oligomer (Weight average molecular weight: 2,500 to 5,000) obtained by polymerizing the compound represented by Structural Formula 8 was used instead of the compound represented by Structural Formula 8.

### Examples 5 to 8 and Comparative Examples 8 to 14. Manufacture of lithium secondary battery

A positive electrode was manufactured by preparing LiNi_{0.5}Mn_{1.5}O₄ having a particle size of 5 µm as a positive electrode active material, preparing a slurry by mixing the positive electrode active material with a carbon-based conductive material and polyvinylidene fluoride as a binder in a weight ratio of 94:3:3 in N-methyl pyrrolidone (NMP), casting the slurry on an aluminum thin film, drying the slurry in a vacuum oven at 120 °C, and rolling the resultant.

Separately, a negative electrode was manufactured by preparing a negative electrode active material in which artificial graphite and silicon oxide (SiO₂) were mixed in a weight ratio of 9:1, preparing a slurry by mixing 97 parts by weight of the negative electrode active material and 3 parts by weight of styrene butadiene rubber (SBR) with water, casting the slurry on a copper thin film, drying the slurry in a vacuum oven at 130 °C, and rolling the resultant.

A lithium secondary battery was manufactured by interposing a separator consisting of 18-µm polypropylene between the positive electrode and negative electrode obtained above, inserting the resultant into a case, and injecting each of the electrolyte compositions prepared in each of Examples 1 to 4 and Comparative Examples 1 to 7 as shown in Table 2 below.

**[Table 2]**

| | Type of electrolyte composition |
|---|---|
| Example 5 | Electrolyte composition of Example 1 (reference) |
| Example 6 | Electrolyte composition of Example 2 |
| Example 7 | Electrolyte composition of Example 3 |
| Example 8 | Electrolyte composition of Example 4 |
| Comparative Example 8 | Electrolyte composition of Comparative Example 1 |
| Comparative Example 9 | Electrolyte composition of Comparative Example 2 |
| Comparative Example 10 | Electrolyte composition of Comparative Example 3 |
| Comparative Example 11 | Electrolyte composition of Comparative Example 4 |
| Comparative Example 12 | Electrolyte composition of Comparative Example 5 |
| Comparative Example 13 | Electrolyte composition of Comparative Example 6 |
| Comparative Example 14 | Electrolyte composition of Comparative Example 7 |

### Experimental Example 1.

To analyze the form in which the electrolyte composition for a lithium secondary battery is present in a secondary battery, the following experiment was performed on the electrolyte compositions prepared in Example 1 and Comparative Example 1, respectively.

### a) Raman spectroscopy Analysis

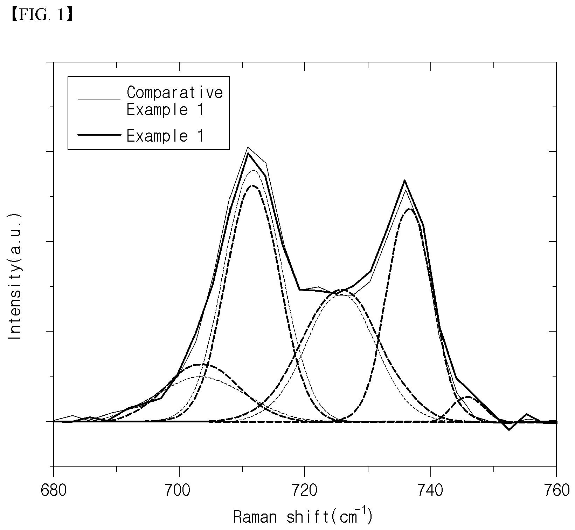

Raman spectroscopy analysis was performed on each electrolyte composition (5 ml) using 532nm laser at a wavelength range of 650 to 760 cm⁻¹. The result is shown in FIG. 1.

Referring to FIG. 1, it was confirmed that, compared with the electrolyte composition prepared in Comparative Example 1 not including an electrolyte additive, the electrolyte composition of Example 1 including the electrolyte additive represented by Formula 8 according to the present invention has an increased Raman spectrum band intensity in the vicinity of 743±1 cm⁻¹. This is a phenomenon that occurs by forming a coordinate bond between ionic materials such as a negatively charged nitrogen atom included in sulfonylimide and a positively-charged lithium ion (Li⁺) of the electrolyte additive represented by Formula 8, indicating that there is an ionic compound in the electrolyte composition of Example 1. In addition, the increase in band intensity indicates that the ionic compound can cause a reduction reaction on the surface of a negative electrode.

### b) Differential capacity curve analysis of half-cell

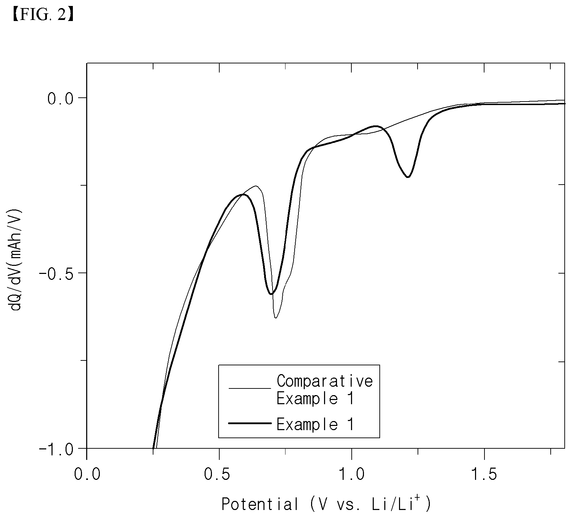

A half-cell was manufactured using lithium metal and graphite (mixture of artificial graphite natural graphite = 9:1 (weight ratio)), and each of the electrolyte compositions prepared in Example 1 and Comparative Example 1 was injected into the half-cell. Afterward, after charging the half-cell at 25 °C from 3.5±0.5V to 0.05V at a rate of 0.005C, a potential value (V) and a capacity value (mAh) were measured, and a reduction potential value was determined by differentiating the capacitance value with respect to the potential value (dQ/dV). The result is shown in FIG. 2.

Referring to FIG. 2, it was confirmed that the electrolyte composition of Example 1 including the electrolyte additive represented by Formula 8 according to the present invention, unlike the electrolyte composition of Comparative Example 1 not including an electrolyte additive, shows a descending peak at a voltage near 1.32V compared to lithium. The descending peak indicates that a reduction reaction occurred on the graphite surface, which is the negative electrode, and the electrolyte additive represented by Formula 8 contained in the electrolyte composition is converted into a coating material through a reduction reaction on the surface of the negative electrode at approximately 1.30V compared to lithium.

### c) Evaluation of linear sweep voltammetry of three-electrode battery

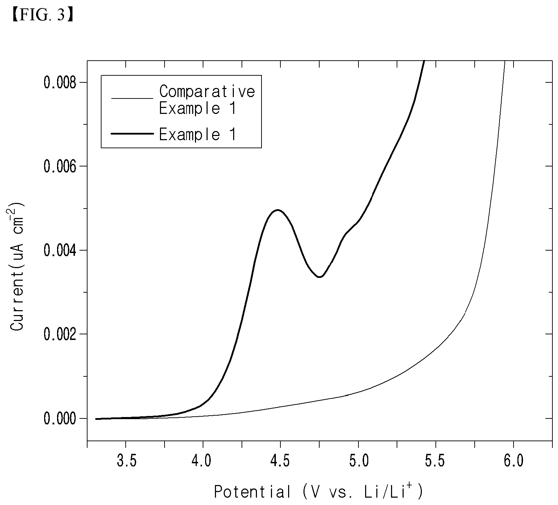

A three-electrode battery was prepared by injecting each of the electrolyte compositions prepared in Example 1 and Comparative Example 1, which includes three electrodes including two platinum electrodes and a lithium metal electrode, and linear sweep voltammetry (LSV) analysis was performed thereon. Here, LSV was performed under the conditions of an observation range of 3.0 to 6.0V (based on lithium), a step voltage of 50 mV and a measurement rate of 50 mV/s. The result is shown in FIG. 3.

Referring to FIG. 3, it can be seen that the electrolyte composition of Example 1 including the electrolyte additive represented by Formula 8 according to the present invention has an increased current near 4.03V compared to lithium. This means that there is an oxidation reaction on the lithium metal surface near 4.03V, and indicates that the electrolyte additive included in the electrolyte composition of Example 1 forms a coating film through an oxidation reaction on the surface of a positive electrode under a condition of 4.03V or more, compared to lithium. In addition, it shows that the oxidation reaction is induced at a lower potential than the surface of the platinum electrode in a carbon electrode or a positive electrode, due to the catalytic properties of carbon or a transition metal.

From the above results, it can be seen that the electrolyte additive according to the present invention is an ionic material, which has an oxidation reaction and a reduction reaction at the positive electrode and the negative electrode, respectively, during charging/discharging of the electrode, thereby forming a coating film on the surface of each electrode.

### Experimental Example 2.

To analyze a coating film formed on an electrode surface in the activation of a lithium secondary battery according to the present invention and evaluate the high-temperature safety of the lithium secondary battery, the following experiment was performed. Here, the targeted lithium secondary batteries as positive electrode active material and negative electrode active material were the secondary batteries of Examples 9 to 12 and Comparative Examples 15 to 21 manufactured in the same manner as in Examples 5 to 8 and Comparative Examples 8 to 14, except that LiNi_{0.6}Co_{0.2}Mn_{0.2}O₂ and artificial graphite were respectively included.

### a) Analysis of coating film on electrode surface

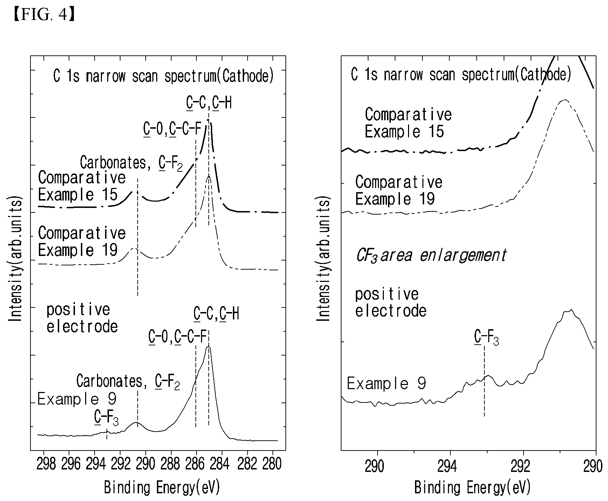

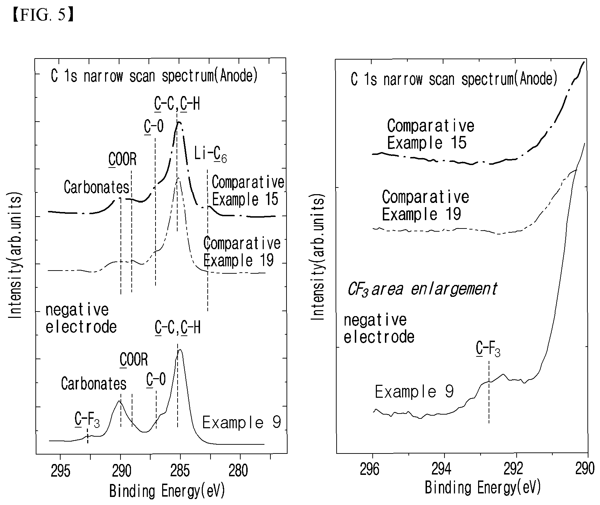

The secondary batteries of Example 9, Comparative Example 15, Comparative Example 19 and Comparative Example 21 were charged/discharged three times at a charge/discharge current density of 0.33C/0.33C, a final charging voltage of 4.2V (NMC/graphite) and a final discharging voltage of 2.5V (NMC/graphite), and X-ray photoelectron spectroscopy (XPS) was performed on the surfaces of the positive and negative electrodes of each battery at full charge. The results are shown in FIGS. 4 and 5.

Referring to FIGS. 4 and 5, it showed that the secondary battery (Example 9) including the electrolyte composition of Example 1 shows peaks in the range of 280 to 300 eV indicating the carbon-fluorine binding energy at the positive and negative electrodes in X-ray photoelectron spectroscopy (XPS) performed on the electrode surface. Specifically, the positive and negative electrodes of the secondary battery showed peaks indicating the binding energy of a CF₃ group at 293±0.2 eV, which means that the electrolyte additive represented by Formula 8 contained in the electrolyte composition is involved in the formation of a coating film formed on the surfaces of the positive and negative electrodes. On the other hand, it was confirmed that the secondary battery (Comparative Example 15) using the electrolyte composition of Comparative Example 1 not including an electrolyte additive, the secondary battery (Comparative Example 19) using the electrolyte composition of Comparative Example 5, and the secondary battery (Comparative Example 21) using the electrolyte composition of Comparative Example 7 do not show the binding energy peak derived from the CF₃ group at both of the positive and negative electrodes.

From the above results, it can be seen that, as the electrolyte additive for a secondary battery according to the present invention includes an ionic compound in the form of monomolecule (or monomer) including a mother nucleus to which a vinyl group is bonded via a function group that extends an amide conjugation or a functional group that has a polyglycolide unit cell at one side based on a sulfonylimide group as shown in any one of Formulae 2-7, 10-28 and 30-35, a coating film containing the ionic compound may be formed on the surface of an electrode in the activation of a secondary battery.

### b) Evaluation of high-temperature storage stability of secondary battery

While each secondary battery was stored at 60 °C for 56 days, ① an amount of gas generation in the secondary battery and ② OCV of the secondary battery were observed over time, and ③ capacity retention rates before and after high-temperature storage were assessed.

Specifically, each secondary battery was charged/discharged three times at a charge/discharge current density of 0.33C/0.33C, a final charging voltage of 4.2V (NMC/graphite), and a final discharging voltage of 2.5V (NMC/graphite) to measure a battery capacity and fully charged with 0.33C at a final charging voltage of 4.2V in a CC/CV mode, followed by starting high temperature storage. Here, the high temperature storage was to store the battery in a constant temperature chamber at 60 °C for a total of 56 days. After 56 days, the OCV deviation (i.e., the degree of OCV drop) and capacity retention rates before and after storage were measured, and the volume of gas generated after high-temperature in the secondary battery was measured using the Archimedes principle. The result is shown in Table 3 below.

**[Table 3]**

| | Type of electrolyte additive used | OCV deviation [mV] | Capacity retention rate [%] | Amount of gas generation [µl] |
|---|---|---|---|---|
| Example 9 reference | 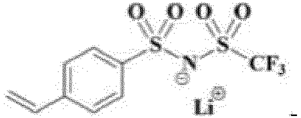 | 32.9 | 97.4 | 1440 |
| Example 10 | 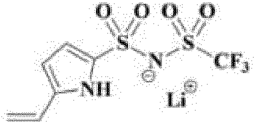 | 33.1 | 98.0 | 1420 |
| Example 11 | 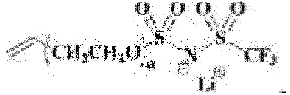 | 33.3 | 97.8 | 1435 |
| Example 12 | 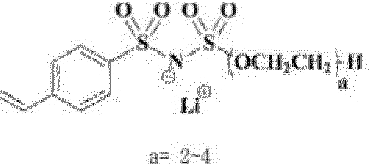 | 33.9 | 97.7 | 1424 |
| Comparative Example 15 | Not added | 38.9 | 95.3 | 1730 |
| Comparative Example 16 | 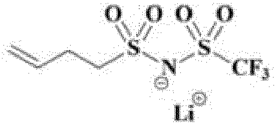 | 40.2 | 94.9 | 2025 |
| Comparative Example 17 | 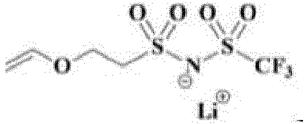 | 39.4 | 95.1 | 1857 |
| Comparative Example 18 | 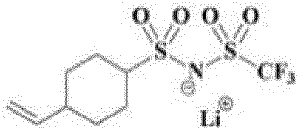 | 39.8 | 94.1 | 1985 |
| Comparative Example 19 | 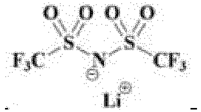 | 38.1 | 95.8 | 1943 |
| Comparative Example 20 | 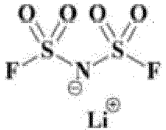 | 39.6 | 94.1 | 1928 |
| Comparative Example 21 | Oligomer derived from <Structural Formula 8> | 39.3 | 95.4 | 1886 |

As shown in Table 3, in the secondary batteries of Examples including the compound represented by any one of Formulae 2-7, 10-28 and 30-35 as an electrolyte additive according to the present invention, it can be seen that, even when being exposed to a high temperature condition, due to the coating film formed on the surfaces of the positive and negative electrodes, the amount of gas generated is significantly reduced by lowering the decomposition of the electrolyte, and the OCV drop phenomenon occurring at the positive electrode decreases.

As above, the present invention has been described with reference to exemplary embodiments, but it should be understood by those killed in the art or those of ordinary skill in the art that the present invention can be variously modified and changed without departing from the spirit and technical scope of the present invention described in the accompanying claims.

Accordingly, the technical scope of the present invention is not limited to the content described in the detailed description of the specification, but should be defined by the claims.

## Claims

1. An electrolyte additive being any one or more compounds selected from Structural Formulae 2-7, 10-28 and 30-35 below:
| | | |
|---|---|---|
| | Structural Formula 2 | Structural Formula 3 |
| | 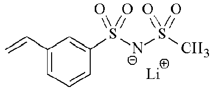 | 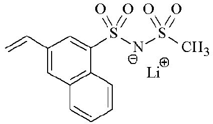 |
| Structural Formula 4 | Structural Formula 5 | Structural Formula 6 |
| 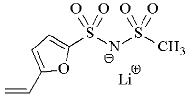 | 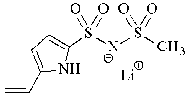 | 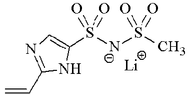 |
| Structural Formula 7 | | |
| 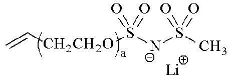 | | |
| Structural Formula 10 | Structural Formula 11 | Structural Formula 12 |
| 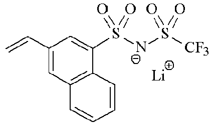 | 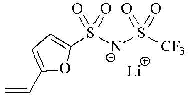 | 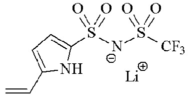 |
| Structural Formula 13 | Structural Formula 14 | Structural Formula 15 |
| 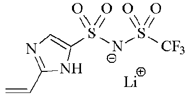 | 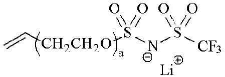 | 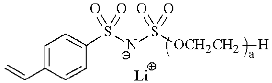 |
| Structural Formula 16 | Structural Formula 17 | Structural Formula 18 |
| 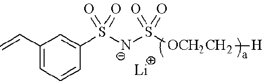 | 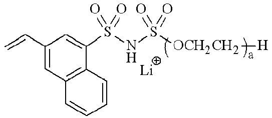 | 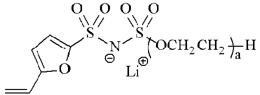 |
| Structural Formula 19 | Structural Formula 20 | Structural Formula 21 |
| 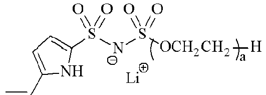 | 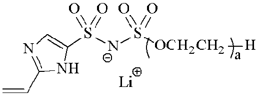 | 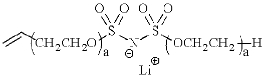 |
| Structural Formula 22 | Structural Formula 23 | Structural Formula 24 |
| 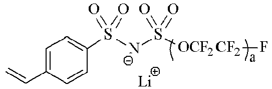 | 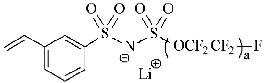 | 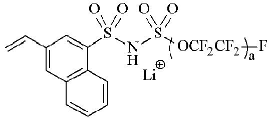 |
| Structural Formula 25 | Structural Formula 26 | Structural Formula 27 |
| 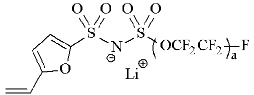 | 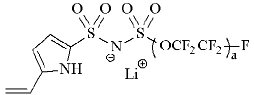 | 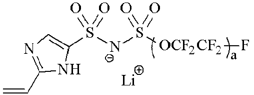 |
| Structural Formula 28 | | Structural Formula 30 |
| 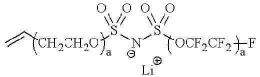 | | 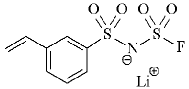 |
| Structural Formula 31 | Structural Formula 32 | Structural Formula 33 |
| 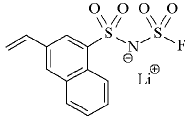 | 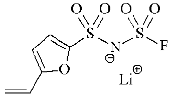 | 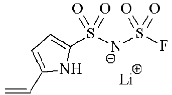 |
| Structural Formula 34 | Structural Formula 35 | |
| 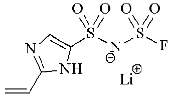 | 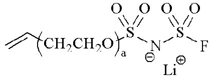 | |
wherein, in Structural Formula 7 and Structural Formula 14 to 28, a is an integer of 2 to 5.

2. An electrolyte composition for a lithium secondary battery, comprising of a non-aqueous organic solvent; lithium salt; and a compound being any one or more compounds selected from Structural Formulae 2-7, 10-28 and 30-35 below:
| | | |
|---|---|---|
| | Structural Formula 2 | Structural Formula 3 |
| | 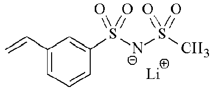 | 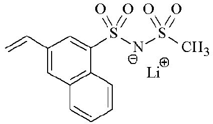 |
| Structural Formula 4 | Structural Formula 5 | Structural Formula 6 |
| 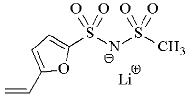 | 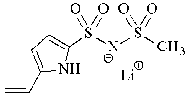 | 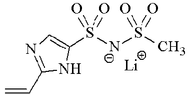 |
| Structural Formula 7 | Structural Formula 8 | Structural Formula 9 |
| 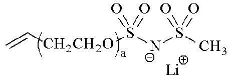 | | |
| Structural Formula 10 | Structural Formula 11 | Structural Formula 12 |
| 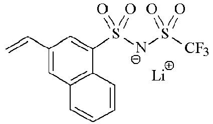 | 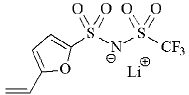 | 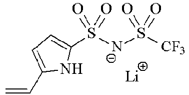 |
| Structural Formula 13 | Structural Formula 14 | Structural Formula 15 |
| 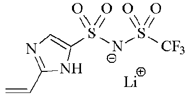 | 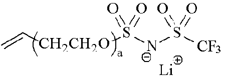 | 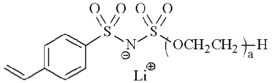 |
| Structural Formula 16 | Structural Formula 17 | Structural Formula 18 |
| 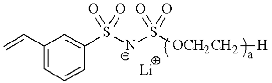 | 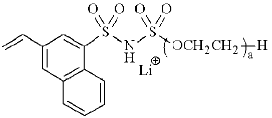 | 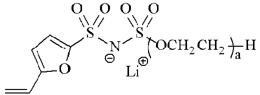 |
| Structural Formula 19 | Structural Formula 20 | Structural Formula 21 |
| 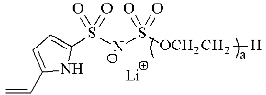 | 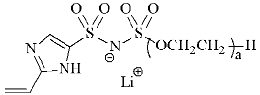 | 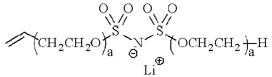 |
| Structural Formula 22 | Structural Formula 23 | Structural Formula 24 |
| 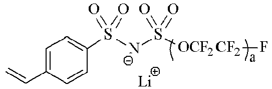 | 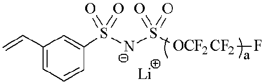 | 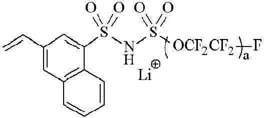 |
| Structural Formula 25 | Structural Formula 26 | Structural Formula 27 |
| 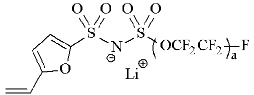 | 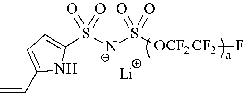 | 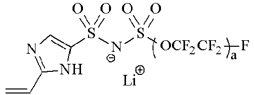 |
| Structural Formula 28 | | Structural Formula 30 |
| 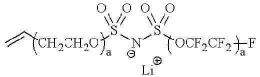 | | 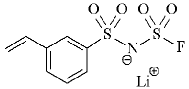 |
| Structural Formula 31 | Structural Formula 32 | Structural Formula 33 |
| 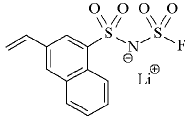 | 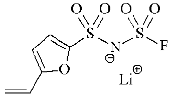 | 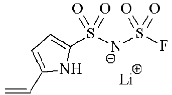 |
| Structural Formula 34 | Structural Formula 35 | |
| 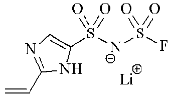 | 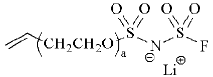 | |
wherein, in Structural Formula 7 and Structural Formula 14 to 28, a is an integer of 2 to 5.

3. The electrolyte composition of claim 2, wherein the compound represented by Formula 1 is comprised at 0.01 to 3wt% with respect to the total weight of the electrolyte composition.

4. The electrolyte composition of claim 2, wherein the lithium salt comprises one or more selected from the group consisting of LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi, and (FSO₂)₂NLi.

5. The electrolyte composition of claim 2, wherein the non-aqueous organic solvent comprises N-methyl-2-pyrrolidinone, ethylene carbonate, propylene carbonate, butylene carbonate, dimethyl carbonate, diethyl carbonate, γ-butyrolactone, 1,2-dimethyoxy ethane, tetrahydrofuran, 2-methyl tetrahydrofuran, dimethyl sulfoxide, 1,3-dioxolane, formamide, dimethylformamide, dioxolane, acetonitrile, nitromethane, methyl formate, methyl citrate, phosphoric acid triester, trimethoxy methane, sulfolane, methyl sulfolane, 1,3-dimethyl-2-imidazolidinone, ether, methyl propionate, and ethyl propionate.

6. A lithium secondary battery, comprising:
an electrode assembly that comprises a positive electrode comprising one or more positive electrode active materials of lithium metal oxides represented by Formulas 2 and 3 below, a negative electrode, and a separator interposed between the positive electrode and the negative electrode, and
the electrolyte composition of claim 2:
[Formula 2] Liₓ[Ni_{y}Co_{z}Mn_{w}M¹ᵥ]O₂
[Formula 3] LiM²ₚMn₍₂₋ₚ₎O₄
In Formulas 2 and 3,
M¹ is one or more elements selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo,
x, y, z, w and v satisfy 1.0≤x≤1.30, 0.5≤y<1, 0<z≤0.3, 0<w≤0.3, and 0≤v≤0.1, respectively, wherein y+z+w+v=1,
M² is Ni, Co or Fe, and
p is 0.05≤p≤0.6.

7. The secondary battery of claim 6, wherein the positive electrode active material comprises one or more selected from the group consisting of LiNi_{0.8}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}Co_{0.2}Mn_{0.15}Al_{0.05}O₂, LiNi_{0.7}Co_{0.1}Mn_{0.1}Al_{0.1}O₂, and LiNi_{0.5}Mn_{1.5}O₄.

8. The secondary battery of claim 6, wherein the negative electrode active material consists of a carbon material and a silicon material, and
the silicon material comprises one or more of silicon (Si), silicon carbide (SiC) and silicon oxide wherein SiO_{q}, 0.8≤q≤2.5.

9. The secondary battery of claim 8, wherein the silicon material is comprised at 1 to 20 wt% with respect to the total weight of the negative electrode active material.

## Patentansprüche

1. Elektrolytadditiv, der eine oder mehrere Verbindungen, ausgewählt aus den folgenden Strukturformeln 2-7, 10-28 und 30-35, ist:
| | | |
|---|---|---|
| | Strukturformel 2 | Strukturformel 3 |
| | 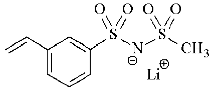 | 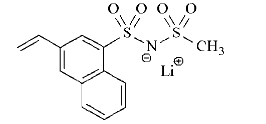 |
| Strukturformel 4 | Strukturformel 5 | Strukturformel 6 |
| 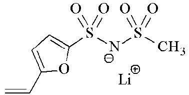 | 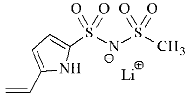 | 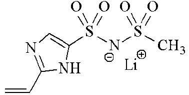 |
| Strukturformel 7 | | |
| 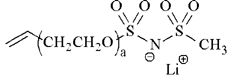 | | |
| Strukturformel 10 | Strukturformel 11 | Strukturformel 12 |
| 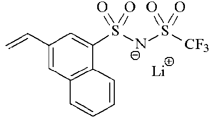 | 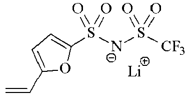 | 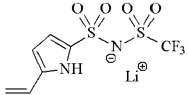 |
| Strukturformel 13 | Strukturformel 14 | Strukturformel 15 |
| 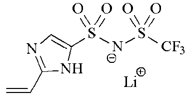 | 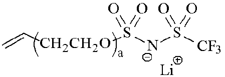 | 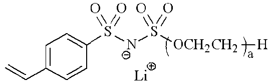 |
| Strukturformel 16 | Strukturformel 17 | Strukturformel 18 |
| 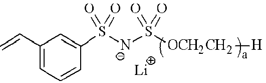 | 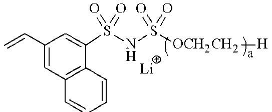 | 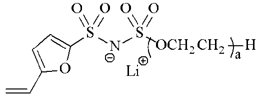 |
| Strukturformel 19 | Strukturformel 20 | Strukturformel 21 |
| 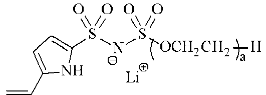 | 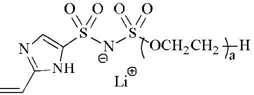 | 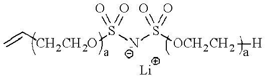 |
| Strukturformel 22 | Strukturformel 23 | Strukturformel 24 |
| 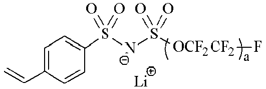 | 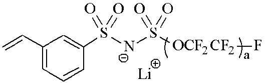 | 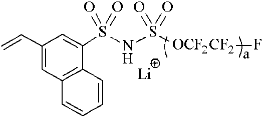 |
| | | |
| Strukturformel 25 | Strukturformel 26 | Strukturformel 27 |
| 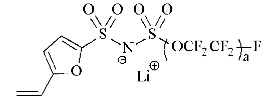 | 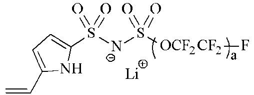 | 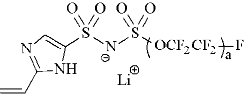 |
| Strukturformel 28 | | Strukturformel 30 |
| 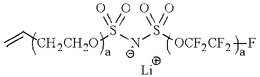 | | 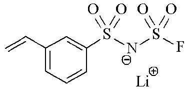 |
| Strukturformel 31 | Strukturformel 32 | Strukturformel 33 |
| 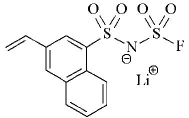 | 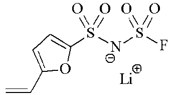 | 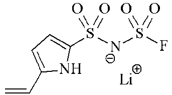 |
| Strukturformel 34 | Strukturformel 35 | |
| 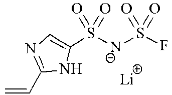 | 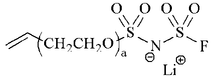 | |
worin, in Strukturformel 7 und Strukturformel 14 bis 28, a eine ganze Zahl von 2 bis 5 ist.

2. Elektrolytzusammensetzung für eine Lithium-Sekundärbatterie, umfassend ein nichtwässriges organisches Lösungsmittel; ein Lithiumsalz; und eine Verbindung, die eine oder mehrere Verbindungen, ausgewählt aus den folgenden Strukturformeln 2-7, 10-28 und 30-35, ist:
| | | |
|---|---|---|
| | Strukturformel 2 | Strukturformel 3 |
| | 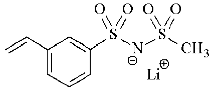 | 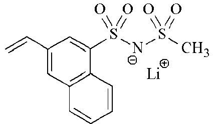 |
| Strukturformel 4 | Strukturformel 5 | Strukturformel 6 |
| 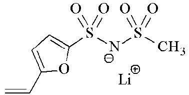 | 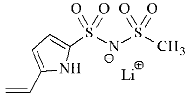 | 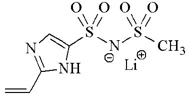 |
| Strukturformel 7 | | |
| 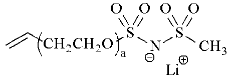 | | |
| Strukturformel 10 | Strukturformel 11 | Strukturformel 12 |
| 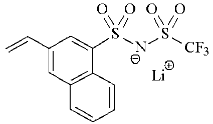 | 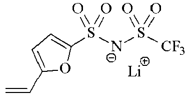 | 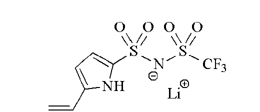 |
| Strukturformel 13 | Strukturformel 14 | Strukturformel 15 |
| 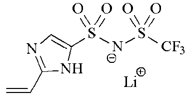 | 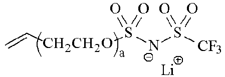 | 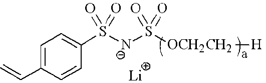 |
| Strukturformel 16 | Strukturformel 17 | Strukturformel 18 |
| 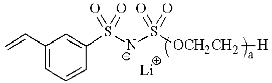 | 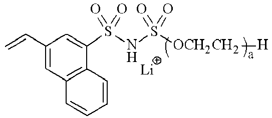 | 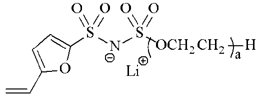 |
| Strukturformel 19 | Strukturformel 20 | Strukturformel 21 |
| 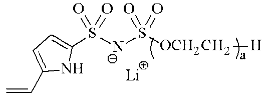 | 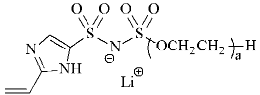 | 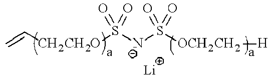 |
| Strukturformel 22 | Strukturformel 23 | Strukturformel 24 |
| 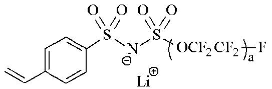 | 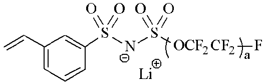 | 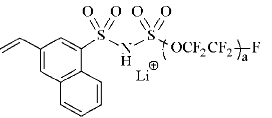 |
| | | |
| Strukturformel 25 | Strukturformel 26 | Strukturformel 27 |
| 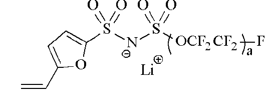 | 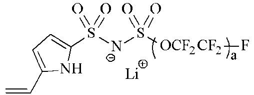 | 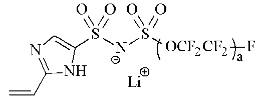 |
| Strukturformel 28 | | Strukturformel 30 |
| 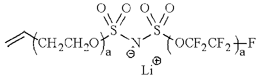 | | 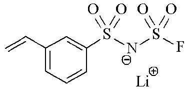 |
| Strukturformel 31 | Strukturformel 32 | Strukturformel 33 |
| 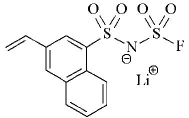 | 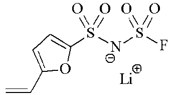 | 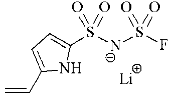 |
| Strukturformel 34 | Strukturformel 35 | |
| 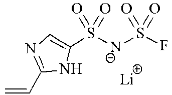 | 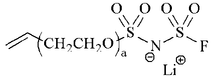 | |
worin, in Strukturformel 7 und Strukturformel 14 bis 28, a eine ganze Zahl von 2 bis 5 ist.

3. Elektrolytzusammensetzung nach Anspruch 2, wobei die durch Formel 1 dargestellte Verbindung in einer Menge von 0,01 bis 3 Gew.-%, bezogen auf das Gesamtgewicht der Elektrolytzusammensetzung, umfasst ist.

4. Elektrolytzusammensetzung nach Anspruch 2, wobei das Lithiumsalz eines oder mehrere umfasst, ausgewählt aus der Gruppe bestehend aus LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi und (FSO₂)₂NLi.

5. Elektrolytzusammensetzung nach Anspruch 2, wobei das nichtwässrige organische Lösungsmittel N-Methyl-2-pyrrolidinon, Ethylencarbonat, Propylencarbonat, Butylencarbonat, Dimethylcarbonat, Diethylcarbonat, γ-Butyrolacton, 1,2-Dimethyoxyethan, Tetrahydrofuran, 2-Methyltetrahydrofuran, Dimethylsulfoxid, 1,3-Dioxolan, Formamid, Dimethylformamid, Dioxolan, Acetonitril, Nitromethan, Methylformiat, Methylcitrat, Phosphorsäuretriester, Trimethoxymethan, Sulfolan, Methylsulfolan, 1,3-Dimethyl-2-imidazolidinon, Ether, Methylpropionat und Ethylpropionat umfasst.

6. Lithium-Sekundärbatterie, umfassend:
eine Elektrodenanordnung, die eine positive Elektrode, die ein oder mehrere Positivelektrodenaktivmaterialien aus Lithiummetalloxiden mit den folgenden Formeln 2 und 3 umfasst, eine negative Elektrode, und einen zwischen der positiven Elektrode und der negativen Elektrode angeordneten Separator umfasst, und
die Elektrolytzusammensetzung nach Anspruch 2:
[Formel 2] Liₓ[Ni_{y}Co_{z}Mn_{w}M¹ᵥ]O₂
[Formel 3] LiM²ₚMn₍₂₋ₚ₎O₄
in den Formeln 2 und 3
ist M¹ ein oder mehrere Elemente, ausgewählt aus der Gruppe bestehend aus W, Cu, Fe, V, Cr, Ti, Zr, Zn, Al, In, Ta, Y, La, Sr, Ga, Sc, Gd, Sm, Ca, Ce, Nb, Mg, B und Mo,
x, y, z, w und v erfüllen jeweils 1,0 ≤ x ≤ 1,30, 0,5 ≤ y < 1, 0 < z ≤ 0,3, 0 < w ≤ 0,3 und 0 ≤ v ≤ 0,1, wobei y + z + w + v = 1 ist,
M² ist Ni, Co oder Fe, und
p ist 0,05 ≤ p ≤ 0,6.

7. Sekundärbatterie nach Anspruch 6, wobei das Positivelektrodenaktivmaterial eines oder mehrere umfasst, ausgewählt aus der Gruppe bestehend aus LiNi_{0.8}Co_{0.1}Mn_{0.1}O₂, LiNi_{0.6}Co_{0.2}Mn_{0.2}O₂, LiNi_{0.9}Co_{0.05}Mn_{0.05}O₂, LiNi_{0.6}Co_{0.2}Mn_{0.1}Al_{0.1}O₂, LiNi_{0.6}Co_{0.2}Mn_{0.15}Al_{0.05}O₂, LiNi_{0.7}Co_{0.1}Mn_{0.1}Al_{0.1}O₂ und LiNi_{0.5}Mn_{1.5}O₄.

8. Sekundärbatterie nach Anspruch 6, wobei das Negativelektrodenaktivmaterial aus einem Kohlenstoffmaterial und einem Siliziummaterial besteht, und
das Siliziummaterial eines oder mehrere aus Silizium (Si), Siliziumcarbid (SiC) und Siliziumoxid, worin SiO_{q}, 0,8 ≤ q ≤ 2,5, umfasst.

9. Sekundärbatterie nach Anspruch 8, wobei das Siliziummaterial zu 1 bis 20 Gew.-%, bezogen auf das Gesamtgewicht des Negativelektrodenaktivmaterials, umfasst ist.

## Revendications

1. Additif électrolytique qui est un ou plusieurs quelconques composés sélectionnés parmi les
Formules Développées 2 à 7, 10 à 28 et 30 à 35 ci-dessous :
| | | |
|---|---|---|
| | Formule Développée 2 | Formule Développée 3 |
| | 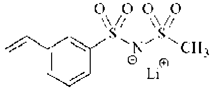 | 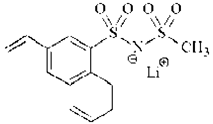 |
| Formule Développée 4 | Formule Développée 5 | Formule Développée 6 |
| 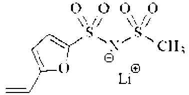 | 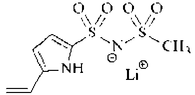 | 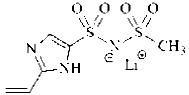 |
| Formule Développée 7 | | |
| 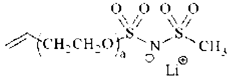 | | |
| Formule Développée 10 | Formule Développée 11 | Formule Développée 12 |
| 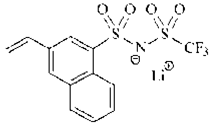 | 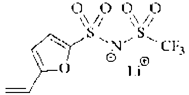 | 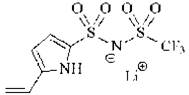 |
| Formule Développée 13 | Formule Développée 14 | Formule Développée 15 |
| 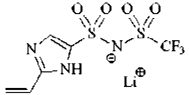 | 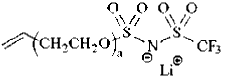 | 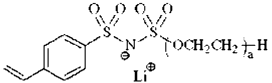 |
| Formule Développée 16 | Formule Développée 17 | Formule Développée 18 |
| 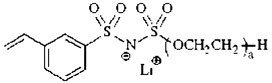 | 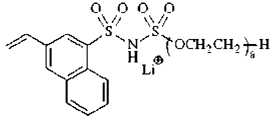 | 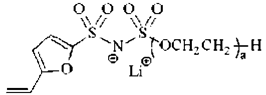 |
| Formule Développée 19 | Formule Développée 20 | Formule Développée 21 |
| 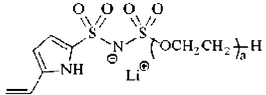 | 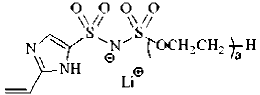 | 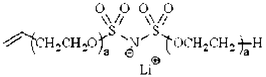 |
| Formule Développée 22 | Formule Développée 23 | Formule Développée 24 |
| 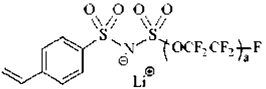 | 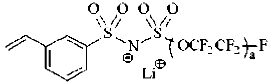 | 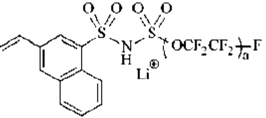 |
| Formule Développée 25 | Formule Développée 26 | Formule Développée 27 |
| 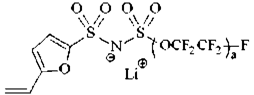 | 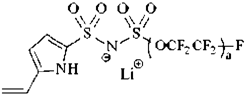 | 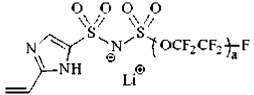 |
| Formule Développée 28 | | Formule Développée 30 |
| 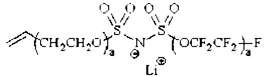 | | 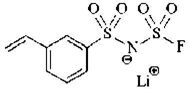 |
| Formule Développée 31 | Formule Développée 32 | Formule Développée 33 |
| 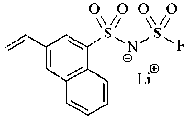 | 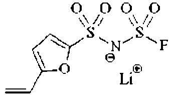 | 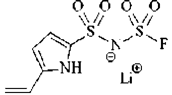 |
| Formule Développée 34 | Formule Développée 35 | |
| 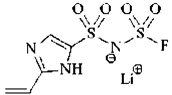 | 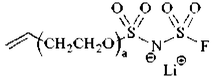 | |
dans lequel, dans la Formule Développée 7 et les Formules Développées 14 à 28, a est un nombre entier de 2 à 5.

2. Composition électrolytique pour une batterie secondaire au lithium, comprenant d'un solvant organique non aqueux ; d'un sel de lithium ; et d'un composé qui est un ou plusieurs quelconques composés sélectionnés parmi les Formules Développées 2 à 7, 10 à 28 et 30 à 35 ci-dessous :
| | | |
|---|---|---|
| | Formule Développée 2 | Formule Développée 3 |
| | 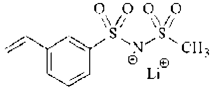 | 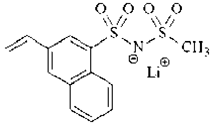 |
| Formule Développée 4 | Formule Développée 5 | Formule Développée 6 |
| 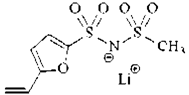 | 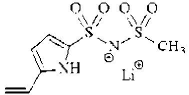 | 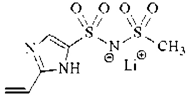 |
| Formule Développée 7 | Formule Développée 8 | Formule Développée 9 |
| 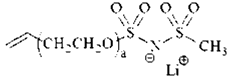 | | |
| Formule Développée 10 | Formule Développée 11 | Formule Développée 12 |
| 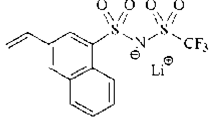 | 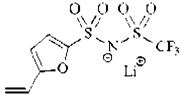 | 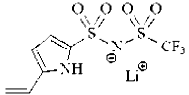 |
| Formule Développée 13 | Formule Développée 14 | Formule Développée 15 |
| 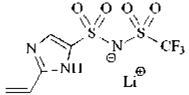 | 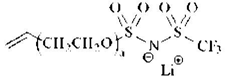 | 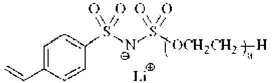 |
| Formule Développée 16 | Formule Développée 17 | Formule Développée 18 |
| 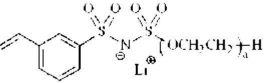 | 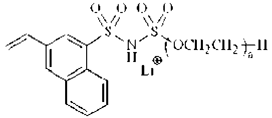 | 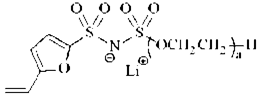 |
| Formule Développée 19 | Formule Développée 20 | Formule Développée 21 |
| 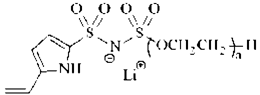 | 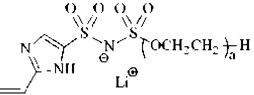 | 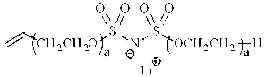 |
| Formule Développée 22 | Formule Développée 23 | Formule Développée 24 |
| 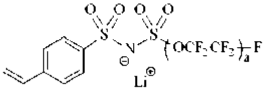 | 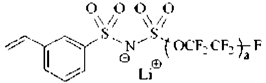 | 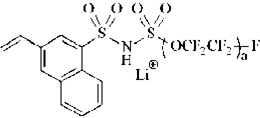 |
| Formule Développée 25 | Formule Développée 26 | Formule Développée 27 |
| 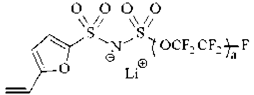 | 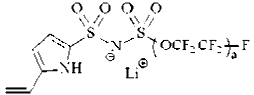 | 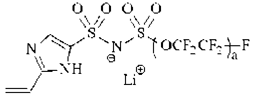 |
| Formule Développée 28 | | Formule Développée 30 |
| 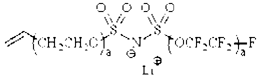 | | 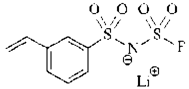 |
| Formule Développée 31 | Formule Développée 32 | Formule Développée 33 |
| 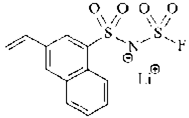 | 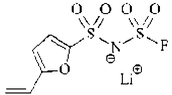 | 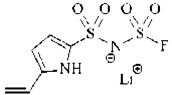 |
| Formule Développée 34 | Formule Développée 35 | |
| 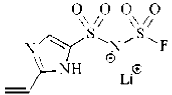 | 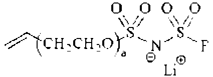 | |
dans laquelle, dans la Formule Développée 7 et les Formules Développées 14 à 28, a est un nombre entier de 2 à 5.

3. Composition électrolytique selon la revendication 2, dans laquelle le composé représenté par la Formule 1 est compris à hauteur de 0,01 à 3 % en poids par rapport au poids total de la composition électrolytique.

4. Composition électrolytique selon la revendication 2, dans laquelle le sel de lithium comprend un ou plusieurs éléments sélectionnés dans le groupe consistant en LiCl, LiBr, Lil, LiClO₄, LiBF₄, LiB₁₀Cl₁₀, LiPF₆, LiCF₃SO₃, LiCF₃CO₂, LiAsF₆, LiSbF₆, LiAlCl₄, CH₃SO₃Li, (CF₃SO₂)₂NLi et (FSO₂)₂NLi.

5. Composition électrolytique selon la revendication 2, dans laquelle le solvant organique non aqueux comprend du N-méthyl-2-pyrrolidinone, du carbonate d'éthylène, du carbonate de propylène, du carbonate de butylène, du carbonate de diméthyle, du carbonate de diéthyle, du γ-butyrolactone, du 1,2-diméthyoxy éthane, du tétrahydrofurane, du 2-méthyl tétrahydrofurane, du sulfoxyde de diméthyle, du 1,3-dioxolane, du formamide, du diméthylformamide, du dioxolane, de l'acétonitrile, du nitrométhane, du formate de méthyle, du citrate de méthyle, de triester d'acide phosphorique, du triméthoxy méthane, du sulfolane, du sulfolane de méthyle, du 1,3-diméthyl-2-imidazolidinone, de l'éther, du propionate de méthyle et du propionate d'éthyle.

6. Batterie secondaire au lithium, comprenant :
un ensemble électrode qui comprend une électrode positive comprenant un ou plusieurs matériaux actifs d'électrode positive d'oxydes métalliques de lithium représentés par les Formules 2 et 3 ci-dessous, une électrode négative et un séparateur interposé entre l'électrode positive et l'électrode négative, et
la composition électrolytique selon la revendication 2 :
[Formule 2] Liₓ[Ni_{y}Co_{z}Mn_{w}M¹ᵥ]O₂
[Formule 3] LiM²ₚMn₍₂₋ₚ₎O₄
dans les Formules 2 et 3,
M¹ est un ou plusieurs éléments sélectionnés dans le groupe consistant en W, Cu, Fe, V, Cr, Ti, Zr, Zn, Al, In, Ta, Y, La, Sr, Ga, Sc, Gd, Sm, Ca, Ce, Nb, Mg, B et Mo,
x, y, z, w et v satisfont 1,0≤x≤1,30, 0,5≤y<1, 0<z≤0,3, 0<w≤0,3 et 0≤v≤0,1, respectivement, dans lesquelles y+z+w+v=1,
M² est Ni, Co ou Fe, et
p est 0,05≤p≤0,6.

7. Batterie secondaire selon la revendication 6, dans laquelle le matériau actif d'électrode positive comprend un ou plusieurs éléments sélectionnés dans le groupe consistant en LiNi_{0,8}Co_{0,1}Mn_{0,1}O₂, LiNi_{0,6}Co_{0,2}Mn_{0,2}O₂, LiNi_{0,9}Co_{0,05}Mn_{0,05}O₂, LiNi_{0,6}Co_{0,2}Mn_{0,1}Al_{0,1}O₂, LiNi_{0,6}Co_{0,2}Mn_{0,15}Al_{0,05}O₂, LiNi_{0,7}Co_{0,1}Mn_{0,1}Al_{0,1}O₂ et LiNi_{0,5}Mn_{1,5}O₄.

8. Batterie secondaire selon la revendication 6, dans laquelle le matériau actif d'électrode négative se compose d'un matériau de carbone et d'un matériau de silicium, et
le matériau de silicium comprend un ou plusieurs parmi silicium (Si), carbure de silicium (SiC) et oxyde de silicium, dans laquelle SiO_{q}, 0,8≤q≤2,5.

9. Batterie secondaire selon la revendication 8, dans laquelle le matériau de silicium est compris à hauteur de 1 à 20 % en poids par rapport au poids total du matériau actif d'électrode négative.
